# NEW EUROPEAN PATENT SPECIFICATION

(11) **EP 1 318 772 B2**
(45) Date of publication and mention of the opposition decision: **21.12.2011**
(45) Mention of the grant of the patent: 30.11.2005
(21) Application number: 01977156.7
(22) Date of filing: 24.09.2001
(51) Int. Cl.: A61F 2/06

(54) **INTRAVASCULAR STENT**
INTRAVASKULÄRER STENT
STENT INTRAVASCULAIRE

(30) Priority: 23.09.2000 US 235167 P
(43) Date of publication of application: 18.06.2003
(60) Divisional application: 05018644.4
(73) Proprietor: Boston Scientific Scimed, Inc., Maple Grove, MN 55311 (US)
(72) Inventor: Jang, G. David, Redlands, CA 92374 (US)
(74) Representative: Hermann, Gerhard
(86) International application number: PCT/US2001/029814
(87) International publication number: WO 2002/024113

(56) References cited:
- EP-A2- 1 236 445
- WO-A-99/15107
- WO-A-99/38457
- WO-A-99/40876
- WO-A1-98/48735
- WO-A2-02/24109
- WO-A2-02/24112
- US-A- 5 733 303
- US-A- 6 039 756

## Description

### BACKGROUND OF THE INVENTION

### Field of Invention:

This invention relates to intravascular stents in general, and more particularly to intracoronary stents.

### Description of the Related Art:

Intracoronary stents provide intraluminal scaffolding support of the vascular wall after percutaneous angioplasty in which the balloon catheter is used to expand the stenotic vascular lesion. In both the delivery phase and the deployed phase, there are numerous performance factors that can characterize the overall clinical performance of a stent and can be improved.

By the year 2000, the percutaneous balloon angioplasty and stent implant procedures have become the dominant non-surgical revascularization method of the atherosclerotic stenosis, or obstruction, of the vascular lumen, and particularly in the coronary vascular system of the heart. With balloon angioplasty alone and without stents, the restenosis rate after angioplasty has been as high as 25-45% in the first time coronary cases. With stents after balloon angioplasty, the restenosis rate has been reduced significantly. Even so, the restenosis rate after stent implantation is reported to be 15-25% range in coronary arteries, depending on the condition of the stented vessel or the specific stent. An ideal coronary stent is still elusive in the current state of the art commercial products.

Document WO99/15107 discloses a stent having a connecting strut column coupling a first expansion column to a second expansion column. The first expansion column has a plurality of slots, the longitudinal axis of the slots being non-parallel to the longitudinal axis of the stent.

Some of the best selling current, second generation, stents can be divided into two categories. One category is a stent with high flexibility and the other category has full vessel coverage. The flexible stents generally have poor vessel coverage, tissue prolapse, rough surface modulation and increased restenosis rate. On the other hand, a stent with good vessel coverage in the current state of art may not be flexible enough for easy delivery and for highly efficient procedures. This means that a stent with good flexibility and good vessel coverage remains as the unfulfilled gold standard.

To further reduce the restenosis rate after stent implant, numerous means have been tried including laser, atherectomy, high frequency ultrasound, radiation device, local drug delivery, etc. Although the brachytherapy (radiation treatment) has proved to be reasonably effective in further reducing restenosis after stent implant, using brachytherpy is very cumbersome, inconvenient, and costly. Brachytherapy is a radioactive device and a radiation therapy specialist from another department has to be involved with the interventional cardiologist in the cardiac catheterization laboratory. The laser and atherectomy devices proved to be marginally useful with added costs.

Local drug therapy appears to be a very promising method for the future, as better pharmaceutical, chemical, or biogenetic agents are developed and became available. Some research data, both from animal tests and human clinical studies indicate evidence of some suppression of restenosis after stent implantation when certain growth blocking or pharmaceutical agents coated the stent. In other instances, it has been speculated that certain surface modifying materials coated on the surface of the stent may be beneficial, alone or in combination with growth suppressing agents, in reducing the restenosis rate. In either instance, a drug or substance should be locally attached or coated on the stent in sufficient amounts. However, attaching or coating a sufficient amount of a substance or drug on the coronary stent may not be an easy proposition, because coating enough volume of the drug on the small surface area of a stent is a challenging task. If and when stent coating becomes practical, a good stent can still have better outcomes than a poorly designed stent when used with substance coating.

A stent is a scaffolding device. When delivered to a remote vessel location via percutaneous approach it can be deployed by expanding the device inside a vessel. The vessel can have a very small caliber and sometimes has a very tortuous anatomy. When a stent is deployed, the stent should have a good radial strength, a good vessel coverage, a good internal surface modulation without tulips (i.e., sharp metal loop projections that resemble fish scale phenomena), an optimal vessel conformability, a low metal fraction, and so forth. If the stent is stiff and non-flexible, it can be very difficult to deliver to an intended lesion site inside a vessel. Easy delivery of a stent is aided by good flexibility of the stent in combination with the delivery balloon, a smooth surface modulation without or minimizing tulips and a degree of radiopacity. A good stent should have a combination of features for delivery and deployment.

Although there are countless variations of vascular stent designs today, few have these desired stent features both in the delivery phase and in the post-delivery phase. Today's top selling stents in the market can have undesirable characteristics, either in the delivery phase or in the deployed phase of the stent life cycle. For example, some stents may have flexibility, but lack vessel coverage or surface modulations both in delivery and deployed phases. Some stents may have good vessel coverage and surface modulations, but lack flexibility.

Vascular stents, which are designed to be delivered to vessel sites via percutaneous approach, can have two elements. The first element is the expansion strut that expands circumferentially to provide the scaffolding radial force against a possible collapsing force of the vessel wall. The second element is the connecting strut that can link the expansion struts along the longitudinal axis of the stent, giving articulation or flexibility to the stent. The particular combination of expansion struts and connecting struts generally form various cells, depending on the specific configuration and shape of the expansion and connecting struts. If a cell is too large, the vessel wall support or coverage can be poor and the vessel wall tissue can prolapse through the large cells of the stent net. If the cells are too small, the vessel wall may be well covered but the metal fraction of the stent can be too high. The metal fraction is a fraction of the total metal surface area of an expanded stent (inside a blood vessel) divided by the total internal vessel wall surface area where the stent is deployed.

Some very flexible stents have very large cell size with poor vessel coverage and tissue prolapse, in addition to poor (inner and/or outer) surface modulation due to large numbers of tulips directed to both ends of the stent. Most of the current flexible stents are designed to effect flexibility by using fewer or a minimal number of connecting struts, handicapping the vessel coverage, surface modulation and tissue prolapse defects.

On the other hand, a stent that is designed for good vessel coverage and ideal cell size tends to be inflexible when such a stent is being delivered to a vessel lesion. A lack of flexibility during stent delivery is a very critical issue; a stiff stent often cannot be delivered to a needed location inside a blood vessel because such a stent cannot navigate through a tortuous and small vessel lumen.

There is a need for a vascular stent that is very flexible for delivery and with good vessel coverage when deployed.

### SUMMARY OF THE INVENTION

Various embodiments of a stent include a combination of maximum possible flexibility and conformability in the stent, full vessel coverage with optimal metal fraction, evenly expanding stent struts, excellent radial strength and radiopacity, and smooth surface modulations in both delivery and deployed phases of the stent life cycle. To arrive at these goals, many innovative new configurations are added to the expansion and connecting strut designs of the stent. Expansion strut design is largely responsible for radial strength and radiopacity, while connecting strut design is largely responsible for flexibility and smooth surface modulations. Full vessel coverage and uniform stent expansion are largely from interaction between expansion and connecting struts. Various embodiments of the stent demonstrate a balance among these multiple qualities, using smart expansion struts and flexible connecting struts in a seamlessly integrated stent network.

The embodiments of the stent are specifically designed to be both very flexible and fully cover vessel surface inside the vascular lumen. The stent can have both characteristics of vessel coverage and flexibility, particularly for coronary use.

Various embodiments of a stent are well designed for both the delivery phase and the deployed phase of the stent life cycle. Both flexibility and good vessel coverage are in a right balance in different embodiments in the stent. Numerous embodiments of the stent include certain configurations in expansion and connecting struts of the stent.

The inventive stent includes a first expansion column, a second expansion column, and a first connecting strut column. The first expansion column and the second expansion column can each include individual expansion struts forming a plurality of expansion strut pairs. Two adjacent expansion strut pairs can share a common strut. The first connecting strut column can include a plurality of individual first connecting struts that couple the first and second expansion columns. Each first connecting strut has a stair-step geometric configuration. The stair-step geometric configuration consists of first and second intermediate sections, a proximal segment coupled to the first expansion column, and a distal segment is a direct extension of an expansion strut of the second expansion column. The invention is further defined by the characterising portion of claim 1.

Some embodiments of the stent, not part of the invention, include a first expansion column, a second expansion column, and a first connecting strut. The first expansion column and the second expansion column can include expansion struts forming a plurality of expansion strut pair loops. Expansion strut pair loops can couple adjacent expansion struts. Two adjacent expansion strut pairs can share a common expansion strut. The first connecting strut column can include a plurality of individual connecting struts. Each first connecting strut has a stair-step geometric configuration with first and second intermediate sections, a proximal segment coupled to the first expansion column, and a distal segment is a direct extension of an expansion strut of the second expansion column.

### BRIEF DESCRIPTION OF DRAWINGS

Figure 1 shows a side elevation view of an embodiment of a stent, such as a tubular stent.
Figure 2 shows an isometric view of an embodiment of a stent, such as a tubular stent.
Figure 3 shows a cut-open view of an embodiment of a stent. Various expansion columns and connecting strut columns are shown.
Figure 4 shows another cut-open view of an embodiment of a stent. Various expansion columns and connecting strut columns are shown.
Figures 5A and 5B show views of expansion struts.
Figures 6A and 6B show more views of expansion struts.
Figure 7A and 7B shows views of connecting struts.
Figure 8 shows a view of conjoining of connecting struts and expansion struts.
Figure 9 shows another view of conjoined connecting struts and expansion struts.
Figure 10 shows another view of conjoined connecting struts and expansion struts.

### DETAILED DESCRIPTION OF DRAWINGS

Some embodiments of stents can be in a state, such as a non-expanded state, an expanded state, a crimped state, and a non-crimped state.

Some embodiments of the stents can include one or more of a first expansion column, a second expansion column, a third expansion column, a first connecting strut column, and a second connecting strut column.

The first expansion column, the second expansion column, and the third expansion column can including individual expansion struts forming a plurality of expansion strut pairs. Two adjacent expansion strut pairs can share a common expansion strut.

The first connecting strut column can include a plurality of individual first connecting struts. First connecting struts conjoin the first and second expansion columns. Each first connecting strut can have a stair-step geometric configuration. The stair-step configuration of a connecting strut can have first and second intermediate sections, a proximal segment coupled to the first expansion column, and a distal segment conjoined directly to an expansion strut of the second expansion column.

The proximal segment of a first connecting strut can be coupled to an associated expansion strut in the first expansion column.

The distal segment of a first connecting strut can be directly extended from an associated expansion strut in the second expansion column.

At least one of a proximal end and a distal end of a first connecting strut can be a direct extension of an expansion strut pair of the first and second expansion columns.

At least one of a proximal end and a distal end of a first connecting strut in the first expansion column can be coupled to an ipsilateral side of an expansion strut pair of the first and second expansion columns, and at a vertical-slant angle to a side of an expansion strut pair of one of the first and second expansion columns.

A longitudinal axis of the distal segment that forms the extension of the associated expansion strut in the second column and a longitudinal axis of the associated expansion strut in the second expansion column are within 20 degrees of each other. The proximal segment of each first connecting strut in the first connecting strut column can be ipsilaterally coupled to an expansion strut pair of the first expansion column and its corresponding distal segment can be ipsilaterally extended from an expansion strut pair of the second expansion column.

A proximal end and a distal end of a first connecting strut in the first connecting strut column can be conjoined on an ipsilateral side of expansion strut pairs of the first and second expansion columns.

Each first connecting strut can have a proximal end that is conjoined to the first expansion column in a first direction, and a distal end that is conjoined to the second expansion column in a second direction that is different from the first direction.

Each first connecting strut has three points of pivot. Each point of pivot can have at least one radius of curvature.

At least a portion of the second intermediate section of a first connecting strut in the first connecting strut column can be positioned in close proximity to a proximal end of an expansion strut pair in the second expansion column. At least a portion of the first intermediate section of a first connecting strut in the first connecting strut column can be in close proximity to a distal end of an expansion strut pair in the first expansion column. Close proximity can be in the range of 0.0254 to 1.27mm (0.001 to 0.050 of an inch).

Each first connecting strut can have a longitudinal axis that is non-parallel to a longitudinal axis of the stent. The longitudinal axis of each first connecting strut can extend in a first direction that is positioned diagonally relative to the longitudinal axis of the stent. Each first connecting strut can have the same longitudinal axis. All of the first connecting struts in a first connecting strut column can have parallel longitudinal axes. At least a portion of the first connecting struts has asymmetrical geometric configurations.

One expansion strut of an expansion strut pair of the first expansion column can have a stair-step segment at a proximal end. The other expansion strut of the expansion strut pair can have a stair-step segment at a distal end. One expansion strut of an expansion strut pair of the second expansion column can have a stair-step segment at a distal end. The other expansion strut of the expansion strut pair can have a stair-step segment at a proximal end.

The first and second intermediate sections of each first connecting strut can be coupled with at least a first radius of curvature. Distal ends of expansion strut pairs of the first expansion column that are coupled to proximal ends of expansion strut pairs of the second expansion column can be vertically offset.

The stent embodiments include a plurality of expansion columns conjoined by a plurality of connecting strut columns.

A plurality of cells is defined by the first expansion column, the second expansion column and the first connecting strut column. The stent cells can have asymmetrical geometries and a quasi-hexagonal geometry in a nominally expanded state.

The second connecting strut column can include a plurality of individual second connecting struts that couple the second and third expansion columns. Each second connecting strut can have a stair-step geometric configuration. The geometric configuration can have first and second intermediate sections, a proximal segment coupled to the first expansion column, and a distal segment conjoined directly to an expansion strut of the second expansion column.

Each second connecting strut can have a longitudinal axis that is non-parallel to a longitudinal axis of the stent. The longitudinal axis of each second connecting strut can extend in a second direction that is positioned diagonally relative to the longitudinal axis of the stent. Each second connecting strut can have the same longitudinal axis. All of the second connecting struts can have parallel longitudinal axes. Each first connecting strut of the first connecting strut column can have a longitudinal axis that extends in a first direction that is opposite to the second direction of the longitudinal axis of the second connecting struts.

Expansion strut pair loops of the first and second expansion columns or second and third expansion columns can be aligned in a peak-to-valley geometry, a valley-to-peak geometry, or a peak-to-peak geometry.

In some embodiments an expansion column includes six cycles of zigzag form made of twelve expansion strut pairs. Each expansion strut pair includes two expansion struts conjoined by a looped joining section at either a proximal or a distal end. This form of pairing of two expansion struts conjoined by a looped joining section alternates between proximal to distal and distal to proximal, continuing twelve times seamlessly around the circumference of an expansion columns in a cylindrically shaped stent. In some embodiments there can be various expansion struts of different types making up twelve blind-loop expansion strut pairs in an expansion column of a cylindrical stent. In order to have twelve blind-loop expansion strut pairs in an expansion column, there also are twelve looped joining sections, half located in proximal ends and half located in distal ends, in an alternating sequence.

There are various forms of expansion column in a stent, some as depicted in Figure-3. Some embodiments include expansion strut columns with pairs of different expansion struts. Some possible expansion struts include a straight expansion strut shape, a straight part with a short step-down segment at a proximal end and a straight part with short step-up segment at a distal end, a straight part with a short step-up segment at a proximal end and a straight part with short step-down segment at a distal end. Other combinations include a longer straight part with a short step-down segment at a proximal end, a longer straight part with a short step-down segment at a distal end, a longer straight part with a short step-up segment at a proximal end, and a longer straight part with a short step-up segment at a distal end. At each step-up or step-down segment, an expansion strut can have a short-sloped transitional section between the long and short parts. Various combinations are within the scope of the invention. On both proximal and distal ends of a stent embodiment in Figure-3, a terminating side of an end expansion column can have smooth and evenly rounded loops.

A step-up or step-down segment can be short in length near a proximal or a distal end and can have a short sloped transitional section. A sloped transitional section can provide flexibility, crimping space, and smooth surface modulation effects to the stent performance. One end of a connecting strut can directly conjoin with the long part of an expansion strut at a sloped transitional section of an expansion strut. Another end of a connecting strut can be conjoined to the side of a short step-up or step-down section. One end of a connecting strut can be a direct extension of an expansion strut on the ipsilateral side. The other end can be laterally conjoined on the ipsilateral side of a step short segment of an expansion strut. A connecting strut is an integral part of the stent structure, rather than a separate structure added, welded or attached. Terminology such as expansion strut or connecting strut conveniently describes the structural anatomy and function of various stent portions.

A connecting strut column, including each connecting strut, has stair-step configurations, for example as shown in Figures-4, -7A & -7B, -8, -9 and - 10. Due to the stair-step configuration, a longitudinal axis of a connecting strut has a diagonal direction to the vertical or horizontal plane of the stent. A connecting strut of the stent can have, in some embodiments, two horizontal sections, two slanted vertical sections, and three pivot points. The horizontal end of a connecting strut can extend from sloped transitional sections of an expansion strut pair loop. The slanted vertical end can conjoin to an ipsilateral side of an apposed expansion strut pair loop at the step down or step-up segment. A stair-step connecting strut can link an expansion strut pair loop of one expansion column to an expansion strut pair loop of an adjacent expansion column in a vertically split-level linking pathway. Each end of a connecting strut can conjoin on the ipsilateral sides of apposing expansion strut pair loops.

A connecting strut that conjoins on ipsilateral sides of expansion strut pair loops, and a split-level linking pathway with multiple pivot points provides stent flexibility, conformability and excellent crimping characteristics to a stent. When each end of a connecting strut is conjoined to a looped pair of expansion struts, the ratio of expansion strut to connecting strut number can be two to one.

When the expansion columns and connecting strut columns are conjoined, the stent can have a continuous, unbroken cylindrical form without breaks or de-linking around the circumference and along the length of a stent. The unbroken link between the expansion and connecting struts can form regular and even asymmetrical cells. The cell size can be maximized or minimized, by programming the dimensions of expansion struts and connecting struts of the stent, as dictated by the clinical or application requirements.

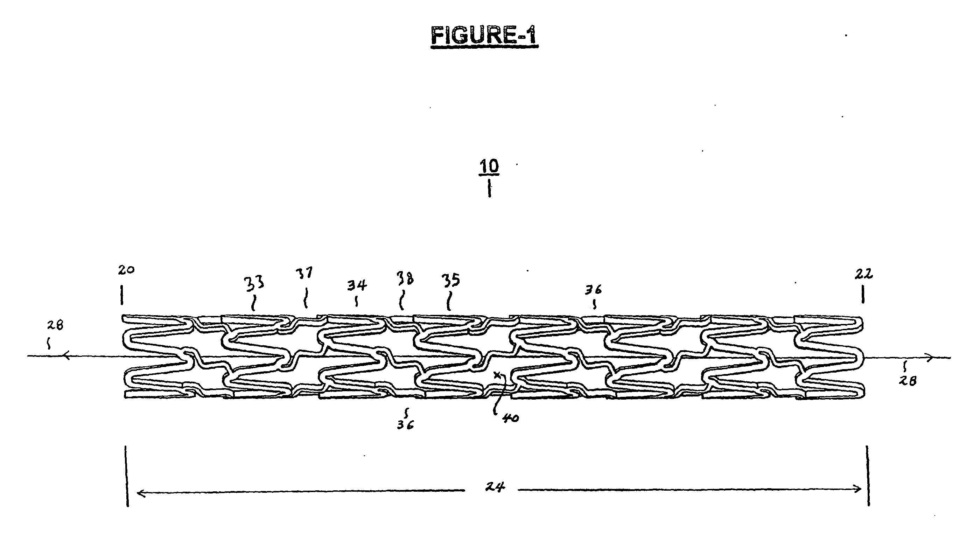

Figure 1 shows one embodiment of a stent 10 in side elevation view. The stent 10 has a proximal end 20 and a distal end 22. The stent 10 can have a tubular or cylindrical structure. The stent 10 can have a longitudinal length 24 and a longitudinal axis 26. Defined by linked connecting and expansion struts are open empty spaces, or cells, for example a cell 40. At both the proximal end 20 and distal end 22 of the stent 10 the terminal ends of expansion strut pairs are evenly rounded for smooth and uniform crimping when the stent 10 is mounted on a delivery balloon.

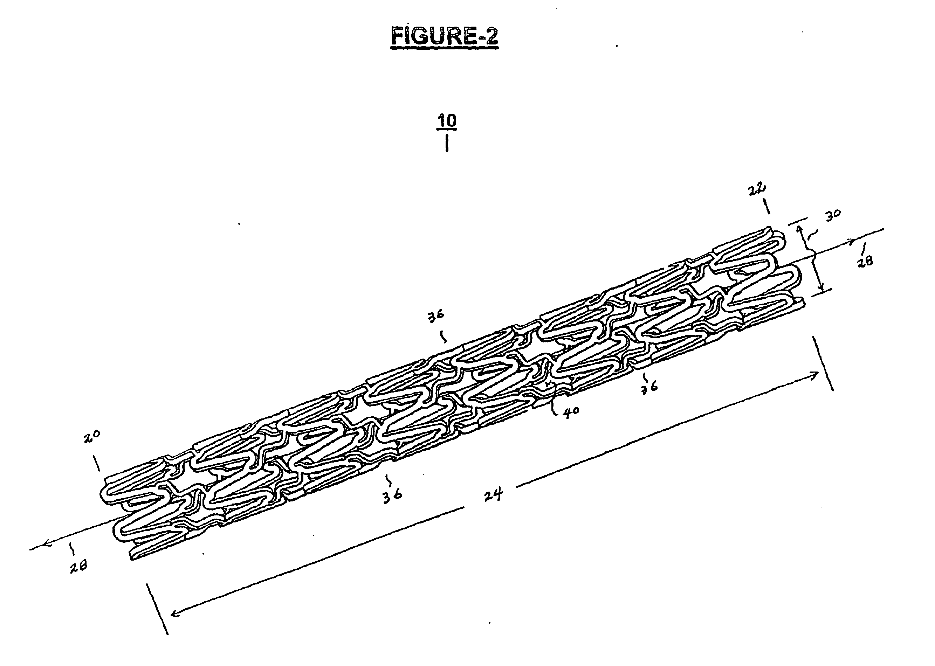

Figure 2 shows an isometric view of an embodiment of a stent, such as a tubular stent. The proximal end 20, distal end 22, the longitudinal axis 26, an internal diameter 30, and a longitudinal length dimension 24 of the cylindrical shape of the stent 10 are shown. The back half of the stent 10 can be seen through cells, such as cell 40.

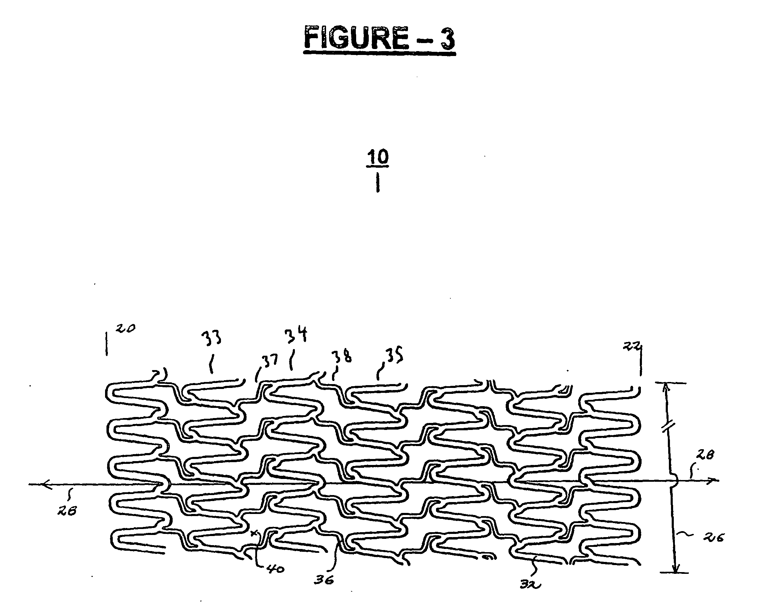

Figure 3 shows a cut-open 2-dimensional view of the cylindrical stent 10. The stent 10 has a longitudinal axis 28, and a circumferential dimension 26. The stent 10 has expansion columns, such as expansion columns 33, 34, and 35; and connecting strut columns, such as connecting strut columns 37 and 38. Expansion column and connecting strut columns alternate in sequence along the longitudinal axis 28 of the stent.

In some embodiment, the expansion columns include a same number of zigzag cycles. Expansion columns shown in Figure-3 have five zigzag cycles. Other embodiments can have more than five cycles, or less than five cycles. In an embodiment with five cycles in expansion columns, there are ten expansion struts. Each cycle includes a pair expansion struts.

Connecting strut columns have stair-step shaped connecting struts, for example connecting strut 36. For every one pair of expansion struts, there is a connecting strut, making for a ratio of expansion struts to connecting struts of two to one.

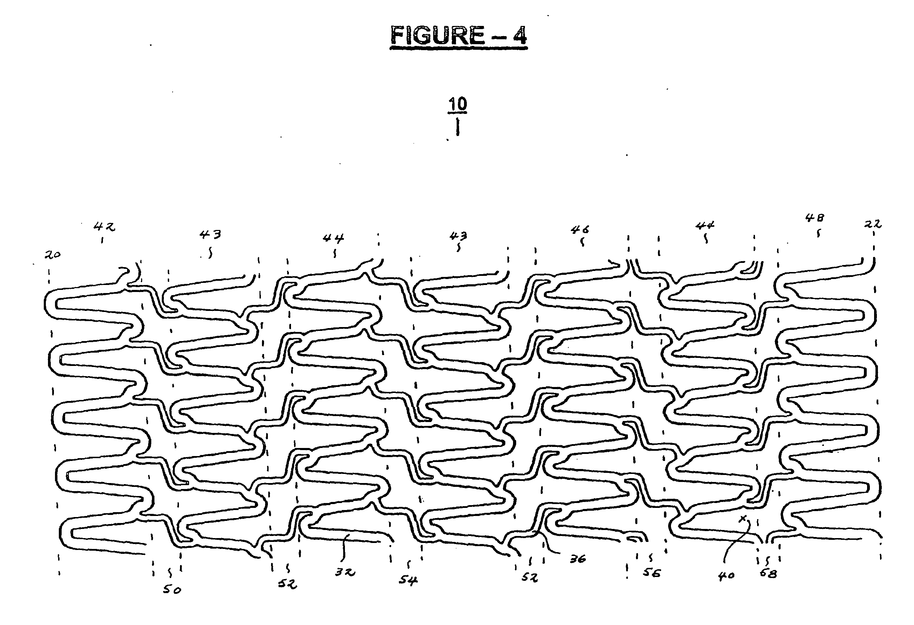

Figure 4 shows expansion columns and connecting strut columns. The embodiment of figure 4 includes a proximal end expansion column 42, a distal end expansion column 48, and expansion columns 43, 44, and 46. In a proximal end expansion column 42, the terminating end loops are rounded in shape making for a smooth surface at the proximal end of the stent 10, suitable for crimping on a delivery balloon. Proximal end expansion column 42 includes an expansion strut type having a straight shape and another expansion strut type having a step-up stair step segment with a sloped-transitional section. These two expansion strut types can make up an expansion strut pair. The distal end expansion strut column 48 can substantially be a mirror image of the proximal end expansion strut column 42. The distal end expansion strut column 48 positions evenly spaced and rounded loops at the terminating end of the stent 10. Evenly spaced and rounded ends can give a smooth surface alignment when the stent 10 is crimped on a delivery balloon.

Expansion column 43 includes different types of expansion strut. One type has a straight strut and the other has a step-up segment at the proximal end and a step-down segment at a distal end. An expansion strut of each type can form an expansion strut pair loop conjoined by a joining strut section, either in the proximal end or the distal end. This pairing can continue around the circumference of the stent 10 in an uninterrupted fashion.

Expansion column 44 includes an expansion strut having a straight shape and another expansion strut with a step-down segment at the proximal end and step-up segment at the distal end. These two types of expansion strut alternate, forming expansion strut pair loops in a continuous manner around the circumference of the cylindrical structure of the stent 10.

Expansion column 46 includes an expansion strut having a step-down segment in the proximal end and an expansion strut having a step-down segment in the distal end. These expansion strut types alternate, forming expansion strut pair loops, in a continuous manner around the circumference.

Figure-4 also shows multiple connecting strut column arrangements. The scope of the invention includes permutations of the expansion column and connecting strut column configurations.

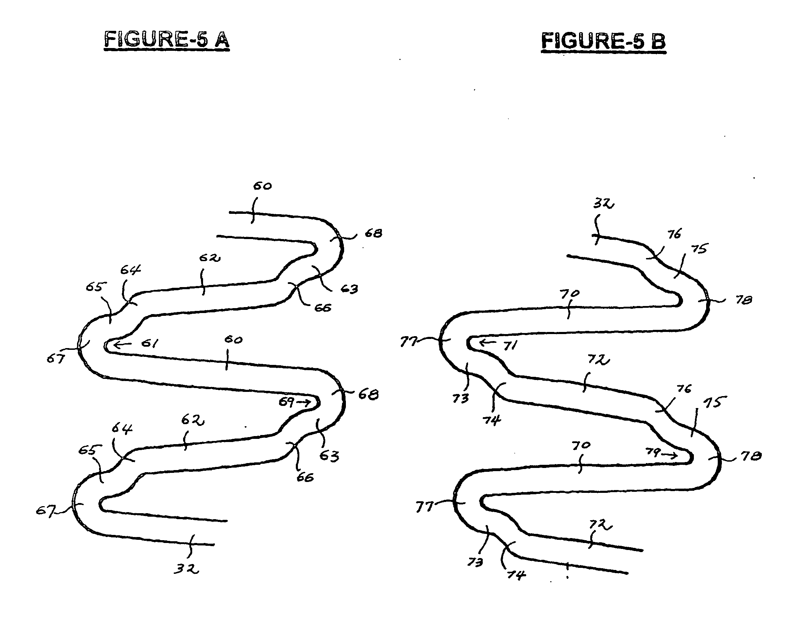

Figure 5A shows expansion struts from expansion column 44. Expansion struts 60 alternate with expansion struts 62, joined either by proximal joining section 67 or distal joining section 68. Proximal joining section 67 defines a proximal cul-de-sac 61. Distal joining section 68 defines a distal cul-de-sac 69. A proximal end of expansion strut 62 includes a step down segment 65 joined by sloped transitional segment 64 to the center of expansion strut 62. A distal end of expansion strut 62 includes a step up segment 63 joined by sloped transitional segment 66 to the center of expansion strut 62.

Figure 5B shows expansion struts from expansion column 43. Expansion struts 70 alternate with expansion struts 72, joined either by proximal joining section 77 or distal joining section 78. Proximal joining section 77 defines a proximal cul-de-sac 71. Distal joining section 78 defines a distal cul-de-sac 79. A proximal end of expansion strut 72 includes a step up segment 73 joined by sloped transitional segment 74 to the center of expansion strut 72. A distal end of expansion strut 72 includes a step up segment 75 joined by sloped transitional segment 76 to the center of expansion strut 72.

Together, Figures 5A and 5B show the valleys of expansion column 44 aligned with the peaks of expansion column 43.

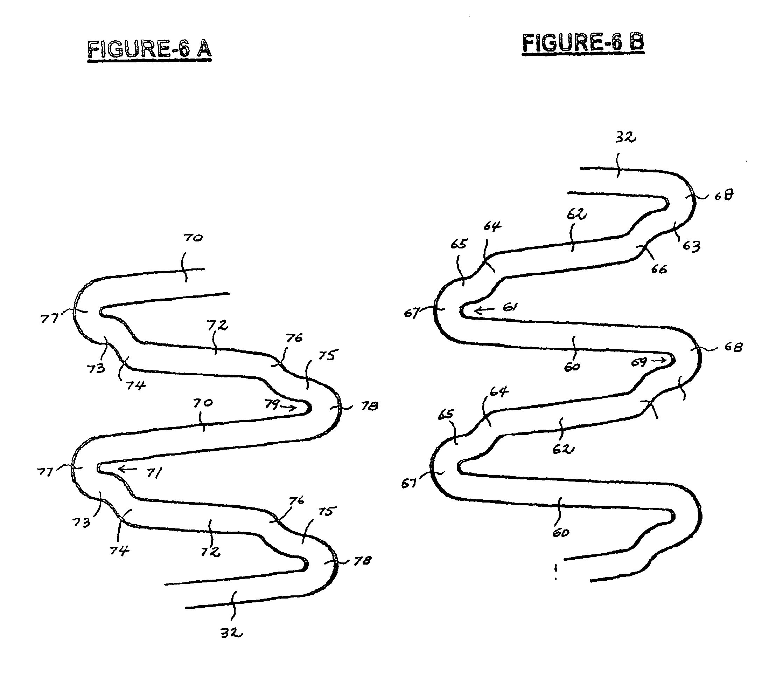

Figure 6A also shows expansion struts of an expansion column 43. Figure 6B also shows expansion struts of an expansion column 44. Together, Figures 6A and 6B shows the valleys of expansion column 43 aligned with the peaks of expansion column 44.

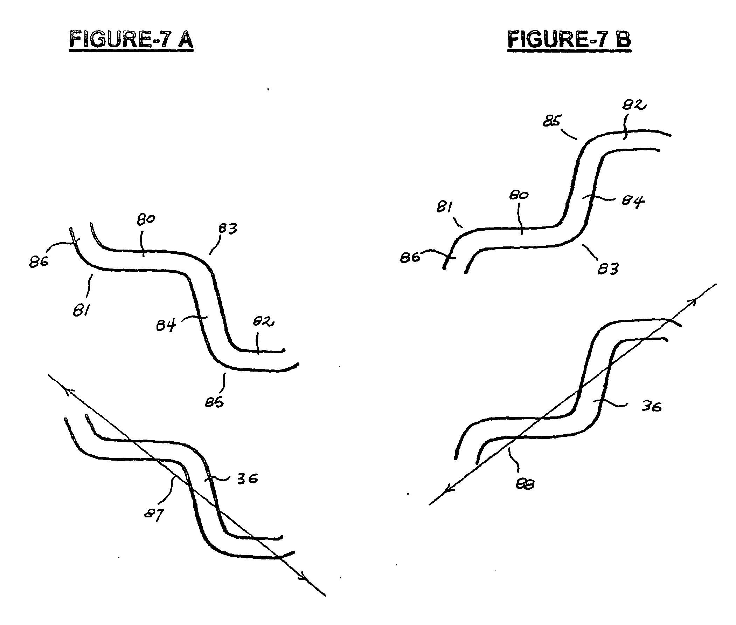

Figure 7A shows connecting struts from connecting strut column 54. A proximal end includes short vertical sloped section 86. Pivot point 81 joins short vertical sloped section 86 with long horizontal section 80. Pivot point 83 joins long horizontal section 80 with long vertical sloped section 84. Pivot point 85 joins long vertical sloped section 84 with short horizontal section 82. Connecting struts in connecting strut column 54 share a longitudinal axis 87.

Figure 7B shows connecting struts from connecting strut column 52, including parts similar to connecting struts from connecting strut column 54. Connecting struts in connecting strut column 52 share a same longitudinal axis 88. The longitudinal axes 87 and 88 are not parallel to each other. Their axes are directed in opposite directions. Figure 7B is a mirror image of Figure 7A.

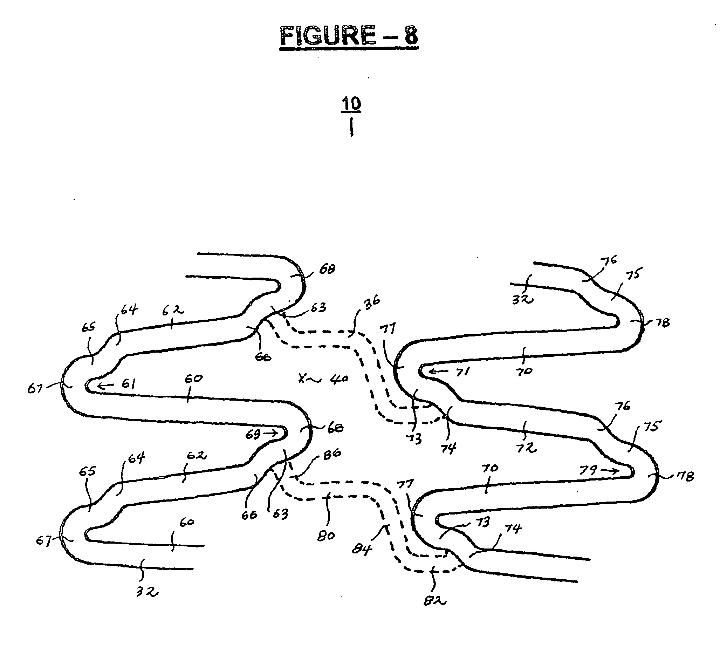

Figure 8 shows the potential conjoining of connecting strut column 54 with expansion strut column 44 on the proximal side of connecting strut column 54 and expansion strut column 43 on the distal side of connecting strut column 54. The step up segment 63 of expansion strut 62 is conjoined to the proximal side of connecting strut column 54. The step up segment 73 of expansion strut 72 is ipsilaterally conjoined to the distal side of connecting strut column 54. The structure of connecting strut column 54 is outlined in dotted lines

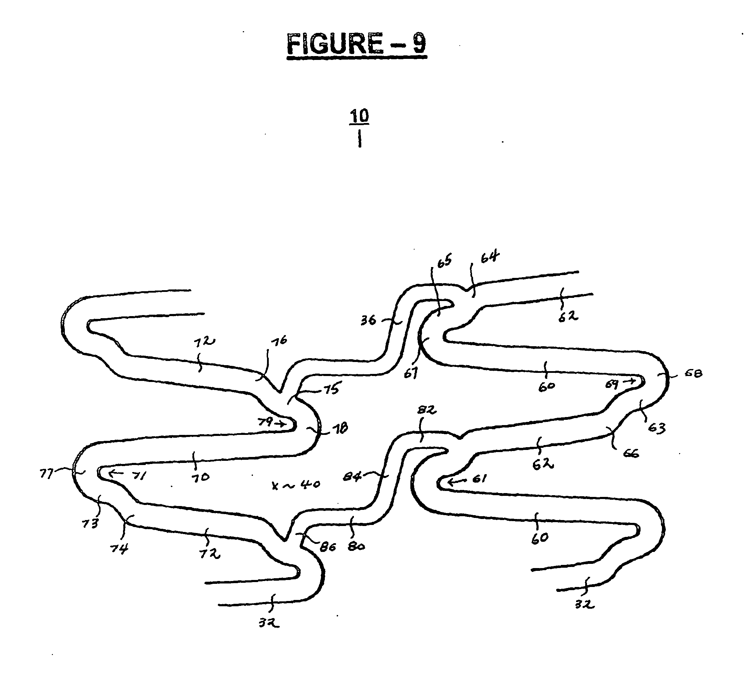

Figure 9 shows the conjoining of connecting strut column 52 with expansion strut column 43 on the proximal side of connecting strut column 52 and expansion strut column 44 on the distal side of connecting strut column 52. The step down segment 75 of expansion strut 72 is conjoined to the proximal side of connecting strut column 52. The step down segment 65 of expansion strut 62 is conjoined to the distal side of connecting strut column 52.

The proximal end 86 of the connecting strut column 52 conjoins on the ipsilateral side to the stepped-down segment 75 of an expansion strut 72. The distal end 82 of a connecting strut column 5 is a direct extension of an expansion strut 62 on the ipsilateral side.

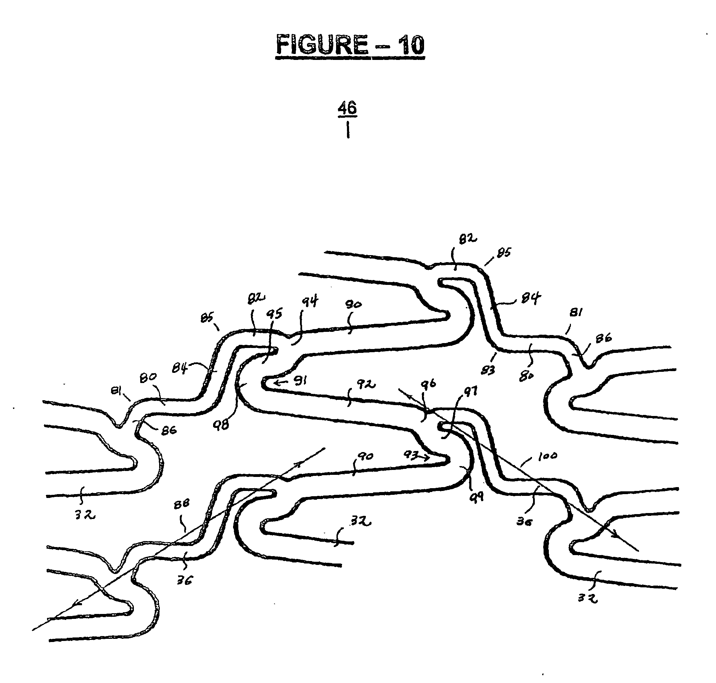

Figure 10 shows the conjoining of expansion column 46 with connecting strut columns 52 and 54. Connecting strut column 52 is conjoined to the proximal side of expansion column 46, and connecting strut column 54 is conjoined to the distal side of expansion strut column 46.

The expansion column 46 shows an important variation of how the connecting struts are conjoined on both proximal and distal ends of the expansion strut pair loops in the expansion strut column 46. Both the proximal expansion strut loops and distal expansion strut loops have direct extensions to the horizontal segment 82 in opposing directions.

## Claims

1. A stent in a non-expanded state, comprising:
a first expansion column (44) including individual : expansion struts (60, 62) forming a plurality of expansion strut pairs, wherein two adjacent expansion strut pairs share a common strut;
a second expansion column (43) including individual expansion struts (70,72) forming a plurality of expansion strut pairs, wherein two adjacent expansion strut pairs share a common strut;
a first connecting strut column (54) including a plurality of individual first connecting struts that couple the first and second expansion columns (44, 43), wherein each of an individual first connecting strut has a stair-step geometric configuration consisting of first and second intermediate sections (80, 84), a proximal segment (86) coupled to the first expansion column (44) and a distal segment (82) conjoined directly to an expansion strut of the second expansion column (43), and
a plurality of expansion columns coupled by a plurality of connecting strut columns;
**characterised in that**:
a longitudinal axis of the distal segment (82) that forms the extension of the associated expansion strut in the first connecting strut column (54) and a longitudinal axis of the associated expansion strut in the second expansion column (43) are within 20 degrees of each other.

2. The stent of claim 1, wherein the proximal segment (86) of a first connecting strut is coupled to an associated expansion strut in the first expansion column (44) and the distal segment (82) of a first connecting strut is directly extended from an associated expansion strut in the second expansion column (43).

3. The stent of claim 1, wherein at least one of the proximal and distal ends of a first connecting strut in the first connecting strut column (54) is a direct extension of an expansion strut pair of one of the first and second expansion columns (44, 43).

4. The stent of claim 1, wherein at least one of the proximal and distal ends of a first connecting strut in the first expansion column is coupled to a side of an expansion strut pair of one of the first and second expansion columns (44, 43), preferably at least one of the proximal and distal ends of a first connecting strut in the first connecting strut column (54) is coupled at a vertical-slant angle to a side of an expansion strut pair of one of the first and second expansion columns (44, 43).

5. The stent of claim 1, wherein the proximal segment (86) of each first connecting strut in the first connecting strut column (54) is ipsilaterally coupled to an expansion strut pair of the first expansion column (44) and its corresponding distal segment (82) is ipsilaterally extended from an expansion strut pair of the second expansion column (43).

6. The stent of claim 1, wherein a proximal end and a distal end of a first connecting strut in the first connecting strut column (54) are conjoined on an ipsilateral side of expansion strut pairs of the first and second expansion columns (44,43).

7. The stent of claim 1, wherein each of a first connecting strut in the first connecting strut column (54) has a proximal end that is conjoined to the first expansion column (43) in a first direction, and a distal end that is conjoined to the second expansion column (44) in a second direction that is different from the first direction.

8. The stent of claim 1, wherein each first connecting strut of the first connecting strut column (54) has three points of pivot (81, 83, 85), preferably each point of pivot has at least one radius of curvature.

9. The stent of claim 1, wherein at least a portion of the first or second intermediate section (80, 84) of a first connecting strut in the first connecting strut column (54) is positioned in close proximity to a proximal end of an expansion strut pair in the second expansion column (43); or
at least a portion of the first or second intermediate section of a first connecting strut (80, 84) in the first connecting strut column (54) is positioned in close proximity to a distal end of an expansion strut pair in the first expansion column (44) preferably close proximity is in the range of 0.0254 to 1.27mm (0.001 to 0.050 of an inch).

10. The stent of claim 1, wherein each first connecting strut of the first connecting strut column (54) has a longitudinal axis that is non-parallel to a longitudinal axis of the stent (10), preferably the longitudinal axis of each first connecting strut extends in a first direction that is positioned diagonally relative to the longitudinal axis of the stent (10); or
each first connecting strut in the first connecting strut column (54) has the same longitudinal axis; or
all of the first connecting struts in the first connecting strut column (54) have parallel longitudinal axes.

11. The stent of claim 11, wherein at least a portion of the first connecting struts of the first connecting strut column (54) have asymmetrical geometric configurations.

12. The stent of claim 1, wherein one expansion strut of an expansion strut pair of the first expansion column (44) has a stair-step segment at a proximal end and the other expansion strut of the expansion strut pair has a stair-step segment at a distal end; or
one expansion strut of an expansion strut pair of the second expansion column (43) has a stair-step segment at a distal end and the other expansion strut of the expansion strut pair has a stair-step segment at a proximal end.

13. The stent of claim 1, wherein the first and second intermediate sections (80,84) of each first connecting strut in the first connecting strut column (54) are coupled with at least one radius of curvature.

14. The stent of claim 1, wherein distal ends of expansion strut pairs of the first expansion column (44) that are coupled to proximal ends of expansion strut pairs of the second expansion column (43) are vertically offset.

15. The stent of claim 1, wherein the first expansion column (44), the second expansion column (43) and the first connecting strut column (54) define a plurality of cells, preferably the plurality of cells have asymmetrical geometries; or
have a quasi-hexagonal geometry in a nominally expanded state.

16. The stent of claim 1, further comprising:
a third expansion column (46) including individual expansion struts forming a plurality of expansion strut pairs, wherein two adjacent expansion strut pairs share a common strut;
a second connecting strut column (52) including a plurality of individual second connecting struts that couple the second and third expansion columns (43,46), wherein each of an individual second connecting strut has a stair-step geometric configuration with first and second intermediate sections (80,84), a proximal segment (86) coupled to the second expansion column (43) and a distal segment (82) conjoined directly to an expansion strut of the third expansion column (46).

17. The stent of claim 16, wherein each second connecting strut of the second connecting strut column (52) has a longitudinal axis that is non-parellel to a longitudinal axis of the stent (10), preferably the longitudinal axis of each second connecting strut extends in a second direction that is positioned diagonally relative to the longitudinal axis of the stent (10); or each second connecting strut in the second connecting strut column (52) has the same longitudinal axis, preferably, all of the second connecting struts in the second connecting strut column (52) have parallel longitudinal axes.

18. The stent of claim 17, wherein each first connecting strut of the first connecting strut column (54) has a longitudinal axis that extents in a first direction that is opposite to the second direction of the longitudinal axis of the second connecting struts.

## Patentansprüche

1. Stent, in nicht-expandierter Stellung, der umfasst:
- ein erstes säulenförmiges Expansionsglied (44), das einzelne Expansionsstreben (60, 62) enthält, die eine Vielzahl von Expansionsstrebenpaaren bilden, wobei zwei benachbarte Expansionsstrebenpaare eine gemeinsame Strebe gemeinsam nutzen;
- ein zweites säulenförmiges Expansionsglied (43), das einzelne Expansionsstreben (70, 72) enthält, die eine Vielzahl von Expansionsstrebenpaare bilden, wobei zwei benachbarte Expansionsstrebenpaare eine gemeinsame Strebe gemeinsam nutzen;
- ein erstes verbindendes säulenförmiges Verstrebungsglied (54), das eine Vielzahl von einzelnen ersten Verbindungsstreben enthält, welche das erste und das zweite säulenförmige Expansionsglied (44, 43) miteinander koppeln, wobei jede der einzelnen ersten Verbindungsstreben eine treppenstufenförmige geometrische Struktur bestehend aus ersten und zweiten Zwischenabschnitten (80, 84), einem nahen Segment (86), das an das erste säulenförmige Expansionsglied (44) gekoppelt ist, und einem fernen Segment (82), das unmittelbar mit einer Expansionsstrebe des zweiten säulenförmigen Expansionsgliedes (43) verbunden ist, besitzt, und
- eine Vielzahl säulenförmiger Expansionsglieder, die durch eine Vielzahl von verbindenden säulenförmigen Verstrebungsgliedern miteinander gekoppelt sind;
**dadurch gekennzeichnet ist, dass**
- eine Längsachse des fernen Segments (82), das das Ansatzstück an die angegliederte Expansionsstrebe in dem ersten verbindenden säulenförmigen Verstrebungsglied (54) bildet, und eine Längsachse der angegliederten Expansionsstrebe im zweiten säulenförmigen Expansionsglied (43) innerhalb von 20 Grad zueinander stehen.

2. Stent nach Anspruch 1, wobei das nahe Segment (86) einer ersten Verbindungsstrebe an die angegliederte Expansionsstrebe im ersten säulenförmigen Expansionsglied (44) gekoppelt ist und das ferne Segment (82) einer ersten Verbindungsstrebe sich unmittelbar von der angegliederten Expansionsstrebe im zweiten säulenförmigen Expansionsglied (43) aus erstreckt.

3. Stent nach Anspruch 1, wobei zumindest eines der nahen und fernen Enden der ersten Verbindungsstrebe im ersten verbindenden säulenförmigen Verstrebungsglied (54) ein direktes Ansatzstück an ein Expansionsstrebenpaar eines der ersten und zweiten säulenförmigen Expansionsglieder (44, 43) ist.

4. Stent nach Anspruch 1, wobei zumindest eines der nahen und fernen Enden einer ersten Verbindungsstrebe im ersten säulenförmigen Expansionsglied an eine Seite eines Expansionsstrebenpaares eines der ersten und zweiten säulenförmigen Expansionsglieder (44, 43) angekoppelt ist, und wobei vorzugsweise zumindest eines der nahen und fernen Enden einer ersten Verbindungsstrebe im ersten verbindenden säulenförmigen Verstrebungsglied (54) in einem vertikal-schrägen Winkel an eine Seite eines Expansionsstrebenpaares eines der ersten und zweiten säulenförmiger Expansionsglieder (44, 43) angekoppelt ist.

5. Stent nach Anspruch 1, wobei das nahe Segment (86) einer jeden ersten Verbindungsstrebe im ersten verbindenden säulenförmigen Verstrebungsglied (54) an der gleichen Seite eines Expansionsstrebenpaares des ersten säulenförmigen Expansionsgliedes (44) angekoppelt ist und sein entsprechendes fernes Segment (82) sich von der gleichen Seite eines Expansionsstrebenpaares des zweiten säulenförmigen Expansionsgliedes (43) aus erstreckt.

6. Stent nach Anspruch 1, wobei ein nahes und ein fernes Ende einer ersten Verbindungsstrebe im ersten verbindenden säulenförmigen Verstrebungsglied (54) auf der gleichen Seite von Expansionsstrebenpaaren der ersten und zweiten säulenförmigen Expansionsglieder (44, 43) und mit diesen verbunden sind.

7. Stent nach Anspruch 1, wobei jede der ersten Verbindungsstreben im ersten verbindenden säulenförmigen Verstrebungsglied (54) ein nahes Ende besitzt, das mit einem ersten säulenförmigen Expansionsglied (44) in einer ersten Richtung verbunden ist, und ein fernes Ende besitzt, das mit einem zweiten säulenförmigen Expansionsglied (43) in einer zweiten Richtung verbunden ist, die von der ersten Richtung verschieden ist.

8. Stent nach Anspruch 1, wobei jede erste Verbindungsstrebe des ersten verbindenden säulenförmigen Verstrebungsgliedes (54) drei Wendepunkte besitzt (81, 83, 85), wobei vorzugsweise jeder Wendepunkt zumindest einen Krümmungsradius aufweist.

9. Stent nach Anspruch 1, wobei zumindest ein Teil des ersten oder zweiten Zwischenabschnitts (80, 84) einer ersten Verbindungsstrebe im ersten verbindenden säulenförmigen Verstrebungsglied (54) in enger Nachbarschaft zu einem nahen Ende eines Expansionsstrebenpaares im zweiten säulenförmigen Expansionsglied (43) positioniert ist; oder zumindest ein Teil des ersten oder zweiten Zwischenabschnitts einer ersten Verbindungsstrebe (80, 84) im ersten verbindenden säulenförmigen Verstrebungsglied (54) in enger Nachbarschaft zu einem fernen Ende eines Expansionsstrebenpaares im ersten säulenförmigen Expansionsglied (44) positioniert ist, wobei die enge Nachbarschaft vorzugsweise in einem Bereich von 0.0254 bis zu 1.27 mm (0.001 bis 0.050 inch) liegt.

10. Stent nach Anspruch 1, wobei jede erste Verbindungsstrebe des ersten verbindenden säulenförmigen Verstrebungsgliedes (54) eine Längsachse besitzt, die nicht parallel zur Längsachse des Stents (10) verläuft, und die Längsachse jeder ersten Verbindungsstrebe sich vorzugsweise in eine erste Richtung erstreckt, die relativ zur Längsachse des Stents (10) diagonal positioniert ist; oder
- jede erste Verbindungsstrebe im ersten verbindenden säulenförmigen Verstrebungsglied (54) die gleiche Längsachse aufweist; oder
- alle ersten Verbindungsstreben im ersten verbindenden säulenförmigen Verstrebungsglied (54) parallel verlaufende Längsachsen besitzen.

11. Stent nach Anspruch 1, wobei zumindest ein Teil der ersten Verbindungsstreben des ersten verbindenden säulenförmigen Verstrebungsglieds (54) asymmetrische geometrische Strukturen aufweist.

12. Stent nach Anspruch 1, wobei die eine Expansionsstrebe eines Expansionsstrebenpaares des ersten säulenförmigen Expansionsgliedes (44) ein treppenstufenförmiges Segment an einem nahen Ende besitzt, und die andere Expansionsstrebe des Expansionsstrebenpaares ein treppenstufenförmiges Segment an einem fernen Ende aufweist; oder eine Expansionsstrebe eines Expansionsstrebenpaares des zweiten säulenförmigen Expansionsgliedes (43) ein treppenstufenförmiges Segment an einem fernen Ende besitzt und die andere Expansionsstrebe des Expansionsstrebenpaares ein treppenstufenförmiges Segment an einem nahen Ende aufweist.

13. Stent nach Anspruch 1, wobei die ersten und zweiten Zwischenabschnitte (80, 84) einer jeden ersten Verbindungsstrebe in dem ersten verbindenden säulenförmigen Verstrebungsglied (54) mit zumindest einem Krümmungsradius gekoppelt ist.

14. Stent nach Anspruch 1, wobei entfernte Enden von Expansionsstrebenpaaren des ersten säulenförmigen Expansionsgliedes (44), die mit nahen Enden von Expansionsstrebenpaaren des zweiten säulenförmigen Expansionsgliedes (43) gekoppelt sind, vertikal versetzt sind.

15. Stent nach Anspruch 1, wobei das erste säulenförmige Expansionsglied (44), das zweite säulenförmige Expansionsglied (43) und das erste verbindende säulenförmige Verstrebungsglied (54) eine Vielzahl von Zellen kennzeichnen, wobei die Vielzahl der Zellen vorzugsweise asymmetrische Geometrien aufweist oder eine quasi-hexagonale Geometrie in einem nominal expandierten Zustand besitzt.

16. Stent nach Anspruch 1, der weiter umfasst:
- ein drittes säulenförmiges Expansionsglied (46), das einzelne Expansionsstreben enthält, die eine Vielzahl von Expansionsstrebenpaaren bilden, wobei zwei benachbarte Expansionsstrebenpaare eine gemeinsame Strebe gemeinsam nutzen;
- ein zweites verbindendes säulenförmiges Verstrebungsglied (52), das eine Vielzahl von einzelnen zweiten Verbindungsstreben enthält, welche die zweiten und dritten säulenförmigen Expansionsglieder (43, 46) miteinander koppeln,
- wobei jede der einzelnen zweiten Verbindungsstreben eine treppenstufenförmige geometrische Struktur aufweist, mit ersten und zweiten Zwischenabschnitten (80, 84), einem nahen Segment (86), das mit dem zweiten säulenförmigen Expansionsglied (43) gekoppelt ist, und einem fernen Segment (82), das unmittelbar mit einer Expansionsstrebe des dritten säulenförmigen Expansionsgliedes (46) verbunden ist.

17. Stent nach Anspruch 16, wobei jede zweite Verbindungsstrebe des zweiten verbindenden säulenförmigen Verstrebungsgliedes (52) eine Längsachse aufweist, die nicht parallel zur Längsachse des Stents (10) verläuft, wobei sich vorzugsweise die Längsachse einer jeden zweiten Verbindungsstrebe in eine zweite Richtung erstreckt, die relativ zu der Längsachse des Stents (10) diagonal positioniert ist; oder jede zweite Verbindungsstrebe im zweiten verbindenden säulenförmigen Verstrebungsglied (52) die gleiche Längsachse aufweist, wobei vorzugsweise alle zweiten Verbindungsstreben im zweiten verbindenden säulenförmigen Verstrebungsglied (52) parallel verlaufende Längsachsen besitzen.

18. Stent nach Anspruch 17, wobei jede erste Verbindungsstrebe des ersten verbindenden säulenförmigen Verstrebungsgliedes (54) eine Längsachse besitzt, die sich in eine erste Richtung erstreckt, die der zweiten Richtung der Längsachse der zweiten Verbindungsstreben entgegengesetzt ist.

## Revendications

1. Stent à l'état non déployé, comprenant :
une première colonne de déploiement (44) comprenant des contre-fiches de déploiement individuelles (60, 62) formant une pluralité de paires de contre-fiches de déploiement, dans lequel deux paires de contre-fiches de déploiement adjacentes partagent une contre-fiche commune ;
une deuxième colonne de déploiement (43) comprenant des contre-fiches de déploiement individuelles (70, 72) formant une pluralité de paires de contre-fiches de déploiement, dans lequel deux paires de contre-fiches de déploiement adjacentes partagent une contre-fiche commune ;
une première colonne de contre-fiches de liaison (54) comprenant une pluralité de premières contre-fiches de liaison individuelles qui assemblent les première et deuxième colonnes de déploiement (44, 43), dans laquelle chaque première contre-fiche de liaison individuelle présente une configuration géométrique en escalier constituée d'une première et d'une seconde section intermédiaire (80, 84), d'un segment proximal (86) couplé à la première colonne de déploiement (44) et d'un segment distal (82) directement relié à une contre-fiche de déploiement de la deuxième colonne de déploiement (43), et
une pluralité de colonnes de déploiement couplées par une pluralité de colonnes de contre-fiches de liaison ;
**caractérisé en ce que** :
un axe longitudinal du segment distal (82) qui forme l'extension de la contre-fiche de déploiement associée dans la première colonne de contre-fiches de liaison (54) et un axe longitudinal de la contre-fiche de déploiement associée dans la deuxième colonne de déploiement (43) sont à 20 degrés l'un de l'autre.

2. Stent selon la revendication 1, dans lequel le segment proximal (86) d'une première contre-fiche de liaison est couplé à une contre-fiche de déploiement associée dans la première colonne de déploiement (44) et le segment distal (82) d'une première contre-fiche de liaison est directement étendu à partir d'une contre-fiche de déploiement associée dans la deuxième colonne de déploiement (43).

3. Stent selon la revendication 1, dans lequel au moins une des extrémités proximale et distale d'une première contre-fiche de liaison dans la première colonne de contre-fiches de liaison (54) est une extension directe d'une paire de contre-fiches de déploiement de l'une des première et deuxième colonnes de déploiement (44, 43).

4. Stent selon la revendication 1, dans lequel au moins une des extrémités proximale et distale d'une première contre-fiche de liaison dans la première colonne de déploiement est couplée à un côté d'une paire de contre-fiches de déploiement de l'une des première et deuxième colonnes de déploiement (44, 43), de préférence au moins une des extrémités proximale et distale d'une première contre-fiche de liaison dans la première colonne de contre-fiches de liaison (54) est couplée selon un angle vertical-incliné à un côté d'une paire de contre-fiches de déploiement de l'une des première et deuxième colonnes de déploiement (44, 43).

5. Stent selon la revendication 1, dans lequel le segment proximal (86) de chaque première contre-fiche de liaison dans la première colonne de contre-fiches de liaison (54) est couplé de manière ipsilatérale à une paire de contre-fiches de déploiement de la première colonne de déploiement (44) et son segment distal correspondant (82) est étendu de manière ipsilatérale à partir d'une paire de contre-fiches de déploiement de la deuxième colonne de déploiement (43).

6. Stent selon la revendication 1, dans lequel une extrémité proximale et une extrémité distale d'une première contre-fiche de liaison dans la première colonne de contre-fiches de liaison (54) sont reliées sur un côté ipsilatéral de paires de contre-fiches de déploiement des première et deuxième colonnes de déploiement (44, 43).

7. Stent selon la revendication 1, dans lequel chaque première contre-fiche de liaison dans la première colonne de contre-fiches de liaison (54) présente une extrémité proximale qui est reliée à la première colonne de déploiement (43) dans une première direction, et une extrémité distale qui est reliée à la deuxième colonne de déploiement (44) dans une seconde direction qui est différente de la première direction.

8. Stent selon la revendication 1, dans lequel chaque première contre-fiche de liaison de la première colonne de contre-fiches de liaison (54) présente trois points de pivotement (81, 83, 85), de préférence chaque point de pivotement présente au moins un rayon de courbure.

9. Stent selon la revendication 1, dans lequel au moins une partie de la première ou seconde section intermédiaire (80, 84) d'une première contre-fiche de liaison dans la première colonne de contre-fiches de liaison (54) est positionnée à grande proximité d'une extrémité proximale d'une paire de contre-fiches de déploiement dans la deuxième colonne de déploiement (43) ; ou
au moins une partie de la première ou seconde section intermédiaire d'une première contre-fiche de liaison (80, 84) dans la première colonne de contre-fiches de liaison (54) est positionnée à grande proximité d'une extrémité distale d'une paire de contre-fiches de déploiement dans la première colonne de déploiement (44), de préférence la grande proximité se situe dans une plage allant de 0,0254 à 1,27 mm (0,001 à 0,050 d'un pouce).

10. Stent selon la revendication 1, dans lequel chaque première contre-fiche de liaison de la première colonne de contre-fiches de liaison (54) présente un axe longitudinal qui est non parallèle à un axe longitudinal du stent (10), de préférence l'axe longitudinal de chaque première contre-fiche de liaison s'étend dans une première direction qui est positionnée en diagonale par rapport à l'axe longitudinal du stent (10) ; ou
chaque première contre-fiche de liaison dans la première colonne de contre-fiches de liaison (54) présente le même axe longitudinal ; ou
l'ensemble des premières contre-fiches de liaison dans la première colonne de contre-fiche de liaison (54) présente des axes longitudinaux parallèles.

11. Stent selon la revendication 1, dans lequel au moins une partie des premières contre-fiches de liaison de la première colonne de contre-fiches de liaison (54) présente des configurations géométriques asymétriques.

12. Stent selon la revendication 1, dans lequel une contre-fiche de déploiement d'une paire de contre-fiches de déploiement de la première colonne de déploiement (44) présente un segment en escalier à une extrémité proximale et l'autre contre-fiche de déploiement de la paire de contre-fiches de déploiement présente un segment en escalier à une extrémité distale ; ou
une contre-fiche de déploiement d'une paire de contre-fiches de déploiement de la deuxième colonne de déploiement (43) présente un segment en escalier à une extrémité distale et l'autre contre-fiche de déploiement de la paire de contre-fiches de déploiement présente un segment en escalier à une extrémité proximale.

13. Stent selon la revendication 1, dans lequel les première et seconde sections intermédiaires (80, 84) de chaque première contre-fiche de liaison dans la première colonne de contre-fiches de liaison (54) sont couplées avec au moins un rayon de courbure.

14. Stent selon la revendication 1, dans lequel les extrémités distales des paires de contre-fiches de déploiement de la première colonne de déploiement (44) qui sont couplées aux extrémités proximales des paires de contre-fiches de déploiement de la deuxième colonne de déploiement (43) sont décalées verticalement.

15. Stent selon la revendication 1, dans lequel la première colonne de déploiement (44), la deuxième colonne de déploiement (43) et la première colonne de contre-fiches de liaison (54) définissent une pluralité de cellules, de préférence la pluralité de cellules présente des géométries asymétriques ; ou
présente une géométrie quasi hexagonale dans un état déployé nominal.

16. Stent selon la revendication 1, comprenant en outre :
une troisième colonne de déploiement (46) comprenant des contre-fiches de déploiement individuelles formant une pluralité de paires de contre-fiches de déploiement, dans lequel deux paires de contre-fiches de déploiement adjacentes partagent une contre-fiche commune ;
une seconde colonne de contre-fiches de liaison (52) comprenant une pluralité de secondes contre-fiches de liaison individuelles qui assemblent les deuxième et troisième colonnes de déploiement (43, 46), dans laquelle chaque seconde contre-fiche de liaison individuelle présente une configuration géométrique en escalier avec une première et une seconde section intermédiaire (80, 84), un segment proximal (86) couplé à la deuxième colonne de déploiement (43) et un segment distal (82) directement relié à une contre-fiche de déploiement de la troisième colonne de déploiement (46).

17. Stent selon la revendication 16, dans lequel chaque seconde contre-fiche de liaison de la seconde colonne de contre-fiches de liaison (52) présente un axe longitudinal qui est non parallèle à un axe longitudinal du stent (10), de préférence l'axe longitudinal de chaque seconde contre-fiche de liaison s'étend dans une seconde direction qui est positionnée en diagonale par rapport à l'axe longitudinal du stent (10) ; ou chaque seconde contre-fiche de liaison dans la seconde colonne de contre-fiches de liaison (52) présente le même axe longitudinal, de préférence l'ensemble des secondes contre-fiches de liaison dans la seconde colonne de contre-fiches de liaison (52) présente des axes longitudinaux parallèles.

18. Stent selon la revendication 17, dans lequel chaque première contre-fiche de liaison de la première colonne de contre-fiches de liaison (54) présente un axe longitudinal qui s'étend dans une première direction qui est opposée à la seconde direction de l'axe longitudinal des secondes contre-fiches de liaison.
